# EUROPEAN PATENT SPECIFICATION

(11) **EP 1 674 616 B1**
(45) Date of publication and mention of the grant of the patent: **19.09.2012**
(21) Application number: 06111552.3
(22) Date of filing: 11.11.2003
(51) Int. Cl.: C11D 3/42, D21H 21/30, C09K 11/06, C07D 251/68, D06L 3/12, C07D 251/50, C07D 251/54, D06M 13/358

(54) **Amphoteric fluorescent whitening agents**
Amphoterer fluoreszenter optischer Aufheller
Agents de blanchiment fluorescents amphotères

(30) Priority: 19.11.2002 EP 02405998
(43) Date of publication of application: 28.06.2006
(62) Divisional of application: 03779887.3
(73) Proprietor: BASF SE, 67056 Ludwigshafen (DE)
(72) Inventor: Scheffler, Goetz, 79639, Grenzach-Wyhlen (DE); Rohringer, Peter, 4124, Schönenbuch (CH); Fletcher, Ian John, 4310, Rheinfelden (CH)
(74) Representative: Zumstein, Angela

(56) References cited:
- EP-A- 0 850 934
- WO-A-96/00220
- WO-A-99/42454
- WO-A-02/055646
- DE-A1- 19 920 784
- US-A- 5 976 410
- US-A- 6 165 973

## Description

The present invention relates to the use of amphoteric bis-triazinylaminostilbene fluorescent whitening agents (FWA's) for fluorescent whitening of synthetic or natural organic materials, in particular, paper.

The most commonly used types of fluorescent whitening agent for the fluorescent whitening of paper are those belonging to the class of di-, tetra- or hexasulphonic acid derivatives of bis-triazinylaminostilbenes, which are anionic in nature. Modern paper-making techniques, however, generally employ cationic polymers as assistants, for example, as retention agents or dewatering aids, in particular, during the production of recycling papers, which, most probably contain residual amounts of anionic FWA's. The presence of cationic polymers, however, results in quenching of the fluorescence of anionic FWA's, which is clearly disadvantageous. Consequently, there is a need for a type of FWA, which is not quenched by such polymers and, in addition, is combinable with anionic FWA's.

EP 0 850 934 A 1 discloses triazinylaminostilbene compounds which are useful for improving the UV protection factor and whiteness of textile fibre materials.

US 6,165,973 discloses a fluorescent whitening agent comprising a mixture of triazinylaminostilbene compounds.

US 5,976,410 discloses a fluorescent whitening agent comprising a triazinylaminostilbene compound and a polyhydroxyl compound.

WO 02/055646 discloses an optical brightener composition comprising a mixture of triazinylaminostilbene compounds.

Surprisingly, it has now been found that certain amphoteric FWA's are neither detrimentally affected by the presence of cationic polymers nor by the presence of residual amounts of anionic FWA's and also exhibit excellent whitening properties when applied to paper.

Accordingly, the invention relates to the use of the compound of formula 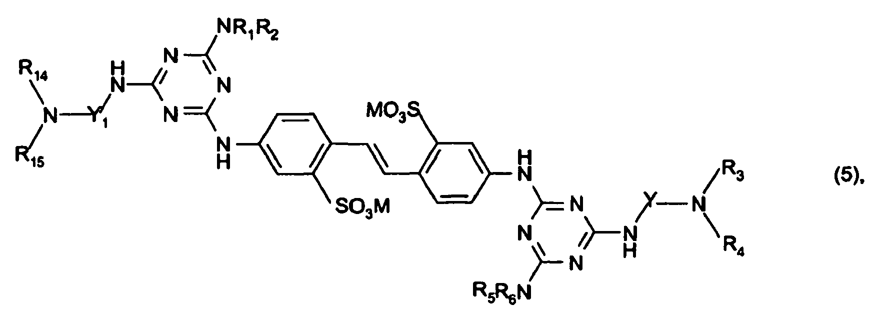 in which
Y and Y₁ each, independently of each other, represent a straight chain C₂-C₈alkylene or branched C₃-C₈alkylene residue, which may be interrupted by one or two nitrogen, oxygen or sulphur atoms or represent a 5- or 6-membered cycloaliphatic ring,
R₁, R₂, R₅ and R₆ each, independently of each other, represent hydrogen, C₁-C₈alkyl, C₂-C₄hydroxyalkyl, C₁-C₄alkoxyC₁-C₄alkyl, phenyl, which is unsubstituted or substituted by halogen, C₁-C₄alkoxy, C₁-C₄alkyl or sulphonamido or
R₁ and R₂ and/or R₅ and R₆, together with the nitrogen atom to which they are attached, complete a morpholino-, piperidino- or pyrrolidino-ring,
R₃, R₄, R₁₄ and R₁₅, each independently of each other, represent hydrogen, C₁-C₄alkyl or C₂-C₄hydroxyalkyl or
R₃ and R₄, together with the nitrogen atom to which they are attached, complete a morpholino-, piperidino- or pyrrolidino-ring and
M represents hydrogen, an alkaline or alkaline earth metal, ammonium or alkylammonium, for the fluorescent whitening of paper.

Preferred compounds of formula (5) are those compounds in which
Y and Y₁ both represent a straight chain C₂-C₆alkylene residue, which may be interrupted by one nitrogen or two oxygen atoms, a branched C₃-C₆alkylene residue or a 1,2-substituted cylohexyl moiety,
R₁, R₂, R₅ and R₆ each represent hydrogen, C₁-C₈alkyl, C₂-C₄ hydroxyalkyl, phenyl, which is unsubstituted or substituted by C₁-C₂alkoxy or sulphonamido or
R₁ and R₂ and/or R₅ and R₆, together with the nitrogen atom to which they are attached, complete a morpholine ring,
R₃, R₄, R₁₄ and R₁₅, each represent hydrogen, C₁-C₄alkyl or C₂-C₄hydroxyalkyl and M represents hydrogen.

Within the scope of the definitions of the substituents, C₁-C₈alkyl groups are, for example, methyl, ethyl, n-propyl, isopropyl, n-butyl, sec-butyl, isobutyl or t-butyl, n-pentyl, ethyl propyl, dimethyl propyl, methyl butyl, n-hexyl, dimethyl butyl, methyl pentyl, ethyl butyl, n-heptyl, methyl hexyl, dimethyl pentyl, ethyl pentyl, trimethyl butyl, n-octyl, methyl heptyl, dimethyl or ethyl hexyl or a trimethyl pentyl, whilst C₁-C₄alkoxy groups are, for example, methoxy, ethoxy, n-propoxy, isopropoxy, n-, sec-, iso- or t-butoxy.

A C₂-C₈alkylene residue, in the definitions of Y and Y₁, may, for example be an ethylene, n-propylene, methyl ethylene, 1- or 2-methylpropylene, n-butylene, ethylethylene, n-pentylene, ethyl propylene, dimethyl propylene, methyl butylene, n-hexylene, dimethyl butylene, methyl pentylene, ethyl butylene, n-heptylene, methyl hexylene, dimethyl pentylene, ethyl pentylene, trimethyl butylene, n-octylene, methyl heptylene, dimethyl or ethyl hexylene or a trimethyl pentylene chain. Where the C₂-C₈alkylene chain is interrupted by heteroatoms, these may be sulphur or, especially, oxygen, whilst C₂-C₄ hydroxyalkyl may be hydroxyethyl, hydroxy-n- or isopropyl or hydroxybutyl.

Further, within the scope of the definitions, halogen is iodine, bromine, fluorine or, especially, chlorine, whilst sulphonamido may be -SO₂NHC₁-C₄alkyl, -SO₂N(C₁-C₄alkyl)₂ or, especially, -SO₂NH₂.

Where M represents an alkaline or alkaline earth metal, this may be lithium, potassium, sodium, calcium or magnesium, whilst alkyl ammonium may be ammonium which is mono-, di-, tri- or tetra substituted by C₁-C₄alkyl or C₂-C₄hydroxyalkyl or a mixture thereof.
Preferably, M represents hydrogen or sodium.

All starting materials are known compounds, which are readily available or may be prepared by known methods.

A further aspect of the invention is a composition for whitening of paper, which contains water, a fluorescent whitening agent of formula (5) and, optionally, auxiliaries.

Such compositions may comprise not only compounds of formula (5) alone, but also mixtures of two or more components with one another.

More specifically, such brightener compositions contain water and, in each case based on the weight of the formulation, from 3 to 25% by weight, preferably from 5 to 15% by weight of the above defined fluorescent whitening agent and also 0 to 60%, preferably 5 to 50% by weight, of auxiliaries.

Suitable auxiliaries include, for example, anionic or non-ionic dispersants from the class of ethylene oxide adducts with fatty alcohols, higher fatty acids or alkyl phenols or ethylenediamine ethylene oxide-propylene oxide adducts, copolymers of N-vinylpyrrolidone with 3-vinylpropionic acid, polyethylene glycols, water retention aids, such as ethylene glycol, glycerol or sorbitol, or biocides.

Since most of the compounds of formula (5) are excellent fluorescent whitening agents for substrates such as paper, the present invention further provides a method for the fluorescent whitening of paper comprising contacting the substrate with a fluorescent whitening agent which comprises a compound of formula (5).

When used for the fluorescent whitening of paper, the compound of formula (5) may be applied to the paper substrate in the pulp mass, in the form of a paper coating composition, or directly in the size press or metering press.

In one preferred aspect, the present invention provides a method for the fluorescent whitening of a paper surface, comprising contacting the paper surface with a coating composition comprising a white pigment; a binder dispersion; optionally a water-soluble co-binder; and sufficient of a fluorescent whitening agent of formula (5) to ensure that the treated paper contains 0.01 to 1 % by weight, based on the white pigment, of a fluorescent whitening agent.

As the white pigment component of the paper coating composition used according to the method of the present invention, there are preferred inorganic pigments, e.g., aluminium or magnesium silicates, such as China clay and kaolin and, further, barium sulfate, satin white, titanium dioxide, calcium carbonate (chalk) or talcum; as well as white organic pigments.

The paper coating compositions used according to the method of the present invention may contain, as binder, inter alia, plastics dispersions based on copolymers of butadiene/styrene, acrylonitrile/butadiene/styrene, acrylic acid esters, acrylic acid esters/styrene/acrylonitrile, ethylene/vinyl chloride and ethylene/vinyl acetate; or homopolymers, such as polyvinyl chloride, polyvinylidene chloride, polyethylene and polyvinyl acetate or polyurethanes. A preferred binder consists of styrene/butyl acrylate or styrene/butadiene/ acrylic acid copolymers or styrene/butadiene rubbers. Other polymer latices are described, for example, in U.S.Patent Specifications 3,265,654, 3,657,174, 3,547,899 and 3,240,740. The optional water-soluble protective colloid may be, e.g., soya protein, casein, carboxymethylcellulose, natural or modified starch, chitosan or a derivative thereof or, especially, polyvinyl alcohol. The preferred polyvinyl alcohol protective colloid component may have a wide range of saponification levels and molecular weights; e.g. a saponification level ranging from 40 to 100; and an average molecular weight ranging from 10,000 to 100,000.

Recipes for coating compositions for paper are described, for example, in J.P. Casey "Pulp and Paper"; Chemistry and Chemical Technology, 2nd edition, Volume III, pages 1684-1649 and in "Pulp and Paper Manufacture", 2nd and 5th edition, Volume II, page497 (McGraw-Hill).

The paper coating compositions used according to the method of the present invention preferably contain 10 to 70% by weight of a white pigment. The binder is preferably used in an amount, which is sufficient to make the dry content of polymeric compound up to 1 to 30% by weight, preferably 5 to 25% by weight, of the white pigment. The amount of fluorescent brightener preparation used according to the invention is calculated so that the fluorescent brightener is preferably present in amounts of 0.01 to 1% by weight, more preferably 0.05 to 1% by weight, and especially 0.05 to 0.6% by weight, based on the white pigment.

The paper coating composition used in the method according to the invention can be prepared by mixing the components in any desired sequence at temperatures from 10 to 100°C, preferably 20 to 80°C. The components here also include the customary auxiliaries, which can be added to regulate the rheological properties, such as viscosity or water retention capacity, of the coating compositions. Such auxiliaries are, for example, natural binders, such as starch, casein, protein or gelatin, cellulose ethers, such as carboxyalkylcellulose or hydroxyalkylcellulose, alginic acid, alginates, polyethylene oxide or polyethylene oxide alkyl ethers, copolymers of ethylene oxide and propylene oxide, polyvinyl alcohol, water-soluble condensation products of formaldehyde with urea or melamine, polyphosphates or polyacrylic acid salts.

The coating composition used according to the method of the present invention is preferably used to produce coated printed or writing paper, or special papers such as ink-jet or photographic papers, or cardboard.

The coating composition used according to the method of the invention can be applied to the substrate by any conventional process, for example with an air blade, a coating blade, a roller, a doctor blade or a rod, or in the size press, after which the coatings are dried at paper surface temperatures in the range from 70 to 200°C, preferably 90 to 130°C, to a residual moisture content of 3-8%, for example with infra-red driers and/or hot-air driers. Comparably high degrees of whiteness are thus achieved even at low drying temperatures.

By the use of the method according to the invention, the coatings obtained are distinguished by optimum distribution of the dispersion of fluorescent brightener over the entire surface and by an increase in the level of whiteness thereby achieved, by a high fastness to light and to elevated temperature (e.g. stability for 24 hours at 60-100°C.) and excellent bleed-fastness to water.

In a second preferred aspect, the present invention provides a method for the fluorescent whitening of a paper surface comprising contacting the paper in the size press with an aqueous solution containing a size, optionally an inorganic or organic pigment and 0.1 to 20g/l of a fluorescent whitening agent of formula (5). Preferably, the size is starch, a starch derivative or a synthetic sizing agent, especially a water-soluble copolymer.

In a third preferred aspect, the invention provides a method for the fluorescent whitening of paper during paper formation, whereby the FWA is added directly to the pulp mass. In this case, the FWA may be in the form of a solution, a dispersion or as a powder, whereby the FWA's of the invention are especially valuable in that their effect is not inhibited by the presence of cationic polymers, fixing agents, wet-strengthening agents or de-inking auxiliaries, which are similarly added to the pulp mass prior to paper formation. Examples of such auxiliaries may include dicyandiamide condensation products, polyvinyl amines, polyethylene imines, cationic starches, poly-DADMAC (diallyl dimethyl ammonium chloride), polyamide amines and polyepoxides.

In a final aspect, the invention relates to paper, which has been treated with a fluorescent whitening agent comprising a compound of formula (5).

The compounds of formula (5) are particularly advantageous in that they exhibit not only extremely high whitening ability, also in the presence of cationic polymers or residual amounts of anionic FWA's, but, in addition, in many cases highly desirable water solubilities and fastness properties.

The following Examples serve to illustrate the invention without intending to be restrictive in nature; parts and percentages are by weight, unless otherwise stated.

### Preparative Examples

### Example 1

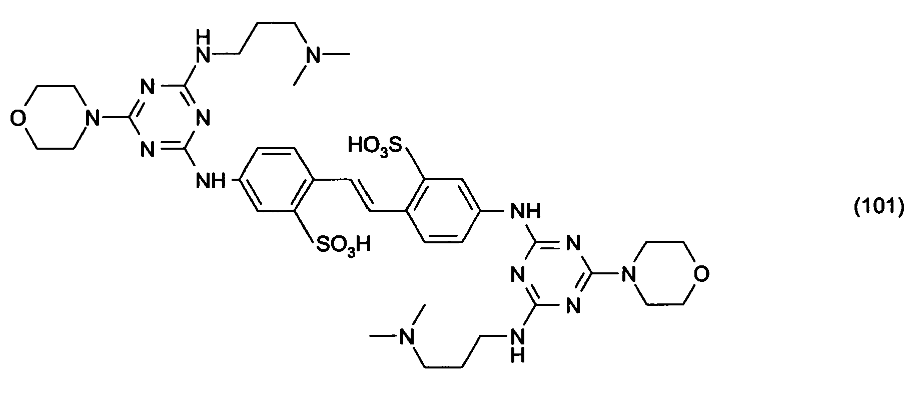

16.7g of 4,4'-bis [(4-N-morpholino-6-chloro-1 ,3,5-triazin-2-yl)amino]stilbene-2,2'-disulphonic acid disodium salt are added over 30 minutes with stirring at 25°C to 50ml of 3-N,N-dimethylamino-1-propylamine, whereby, during the addition, the temperature rises to 60°C. The temperature is then further increased to 100°C and the mixture maintained at this temperature for a further 1 hour. Heating is then ceased, the mixture allowed to stand overnight at room temperature, then diluted with 25ml of water and evaporated under vacuum to approximately 30g. The resulting residue is dissolved in 50ml of water and the pH adjusted to 1.0 by addition of 20ml of concentrated hydrochloric acid. The pH is then raised to approximately 5 and the mixture stirred overnight at room temperature. The precipitated solids are filtered, washed with water and dried under vacuum at 60°C. There are obtained 14.9g of the compound of formula (101) as pale yellow crystals with an active content of 83%.

The starting material, 4,4'-bis [(4-N-morpholino-6-chloro-1,3,5-triazin-2-yl)amino]stilbene-2,2'-disulphonic acid disodium salt of formula (101a), is prepared as follows:
A solution of 120g of cyanuric chloride in 930ml of methyl ethyl ketone is added with stirring over 10 minutes at 5-10°C to 400g of ice/water. Then, during 70 minutes at a pH of from 4.5 to 5.0, 1042g of a 12% solution of 4,4'-diaminostilbene-2,2'-disulphonic acid and sodium carbonate are added such that no excess of 4,4'-diaminostilbene-2,2'-disulphonic acid is present. The mixture is stirred for a further 20 minutes at 5-10°C, after which time a total of 37.9ml of 20% aqueous sodium carbonate solution is consumed. The mixture is warmed to 15-20°C and the pH adjusted to 7.0 by addition of 20% aqueous sodium carbonate solution. 28.0g of morpholine are then added drop wise over 10 minutes, the mixture warmed to 70-75°C during 60 minutes and stirring continued for 30 minutes at this temperature, the pH being maintained at 7.0-7.5 by addition of a total of 46.9ml of 50% aqueous sodium hydroxide solution. The temperature is then raised to 90°C and the methyl ethyl ketone distilled off. The reaction mixture is then slowly cooled to 25°C over 60 minutes, the precipitated solids filtered, washed with 5% brine and dried under vacuum at 60°C. There are obtained 232.2 g of the compound of formula

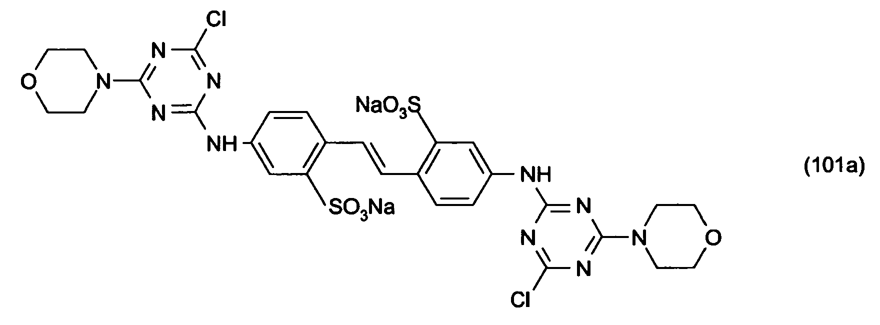

### Example 2

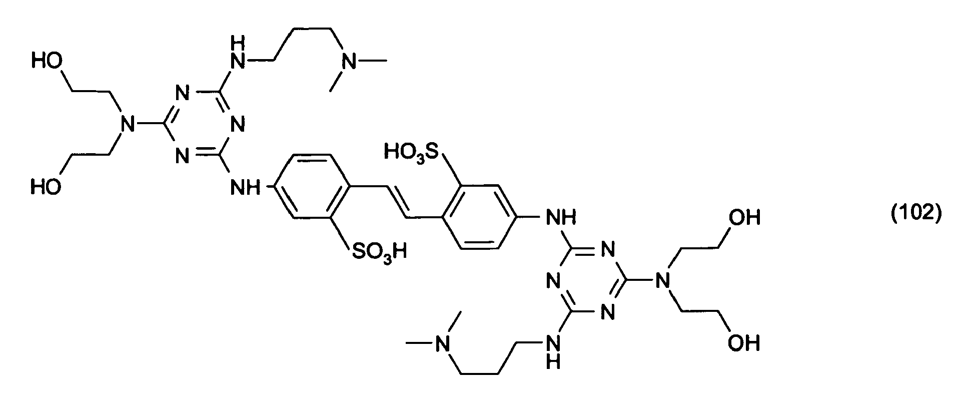

8.9g of 4,4'-bis [(4-bis-(2-hydroxethyl)amino-6-chloro-1,3,5-triazin-2-yl)amino]stilbene-2,2'-disulphonic acid disodium salt (102a), obtained by an analogous process to that described for compound (101a) in Example 1, are added over 10 minutes with stirring at 25°C to 25ml of 3-N,N-dimethylamino-1-propylamine, whereby, during the addition, the temperature rises to 45°C. The temperature is then further increased to 100°C and the mixture maintained at this temperature for a further 1.75 hours. Heating is then ceased, the mixture allowed to stand overnight at room temperature, then diluted with 25ml of water and evaporated under vacuum to approximately 18g. The resulting residue is diluted with 50ml of water and the pH adjusted to 1.0 by addition of aqueous 17% hydrochloric acid. 90ml of acetone are then added, resulting in the formation of 2 phases. The aqueous phase is separated off in a separating funnel and the pH is then raised to 8.5 by addition of 4N aqueous sodium hydroxide solution. The precipitated solids are filtered, washed with water and dried under vacuum at 60°C. There are obtained 5.0g of the compound of formula (102) as yellow crystals.

### Example 3

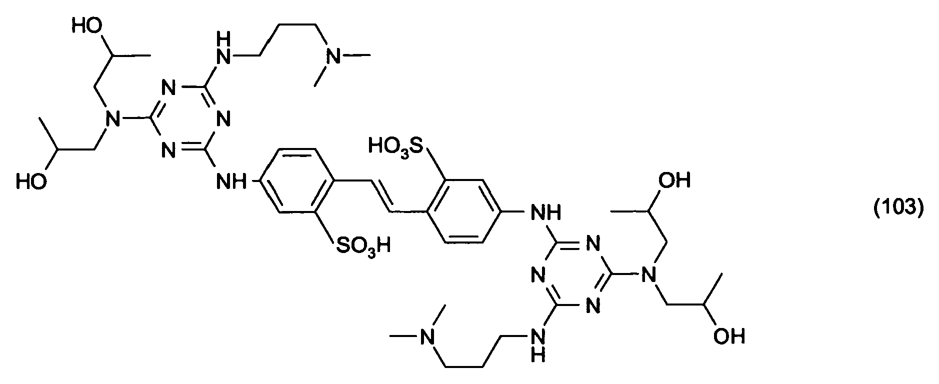

By proceeding essentially as described in Example 2, but replacing the 4,4'-bis [(4-bis(2-hydroxyethyl)amino-6-chloro-1,3,5-triazin-2-yl)amino]stilbene-2,2'-disulphonic acid disodium salt (102a) by an equivalent quantity of 4,4'-bis [(4-bis(2-hydroxy-n-propyl)amino-6-chloro-1,3,5-triazin-2-yl)amino]stilbene-2,2'-disulphonic acid disodium salt (103a), obtained by an analogous process to that described for compound (101a) in Example 1, there are obtained 6.4g of the compound of formula (103) as pale yellow crystals

### Example 4

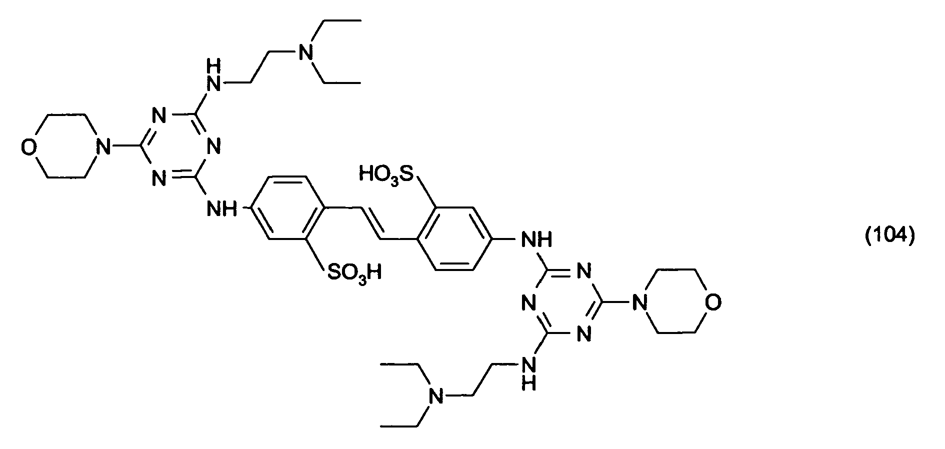

24.3g of 4,4'-bis [(4-N-morpholino-6-chloro-1 ,3,5-triazin-2-yl)amino]stilbene-2,2'-disulphonic acid disodium salt (101a) are added over 15 minutes with stirring at 69.6g of 2-N,N-diethylamino-1-ethylamine. The resulting suspension is then heated to 115°C and the mixture maintained at this temperature for a further 2 hours. After diluting with 150ml of water, the pale brown solution is evaporated under vacuum and this procedure repeated twice. Addition of 100ml of water to the residue results in a beige suspension of pH 11.2. The pH is then adjusted to 12.8 by addition of 5ml of 50% aqueous sodium hydroxide solution, then lowered to 4 by addition of 35ml of concentrated hydrochloric acid, the yellow precipitate stirred for 30 minutes, filtered and washed with 1000ml of water. After drying under vacuum at 70°C, there are obtained 26.7g of the compound of formula (104) as yellow crystals.

### Example 5

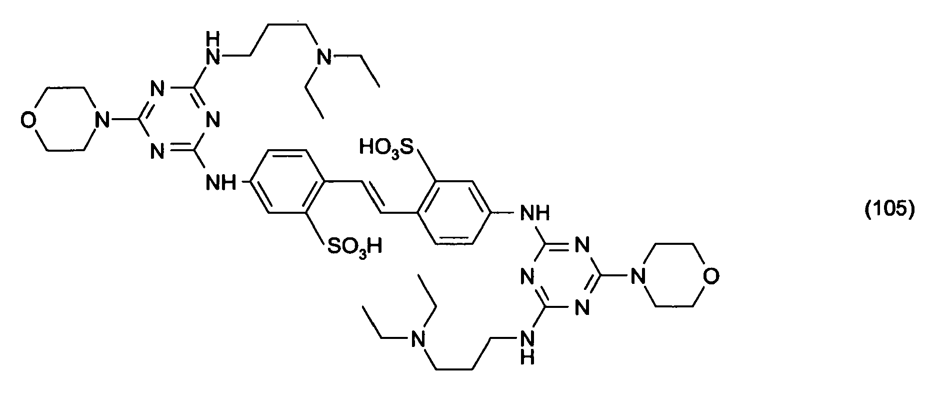

By proceeding essentially as described in Example 4, but replacing the 2-N,N-diethylamino-1-ethylamine by an equivalent quantity of 3-N,N-diethylamino-1-propylamine, there are obtained 27.0g of the compound of formula (105) as whitish beige crystals.

### Example 6

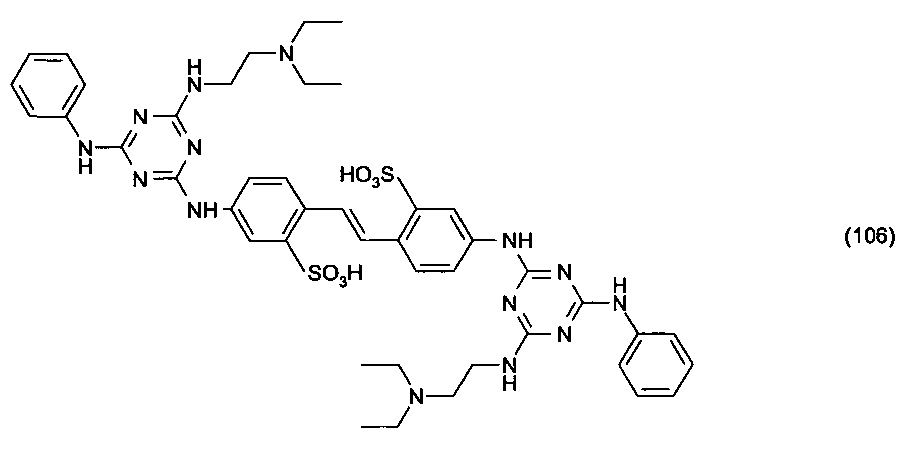

By proceeding essentially as described in Example 4, but replacing the 4,4'-bis [(4-N-morpholino-6-chloro-1,3,5-triazin-2-yl)amino]stilbene-2,2'-disulphonic acid disodium salt by an equivalent quantity of 4,4'-bis [(4-anilino-6-chloro-1,3,5-triazin-2-yl)amino]stilbene-2,2'-disulphonic acid disodium salt (106a), obtained by an analogous process to that described for compound (101a) in Example 1, there are obtained 19.9g of the compound of formula (106) as beige crystals.

### Example 7

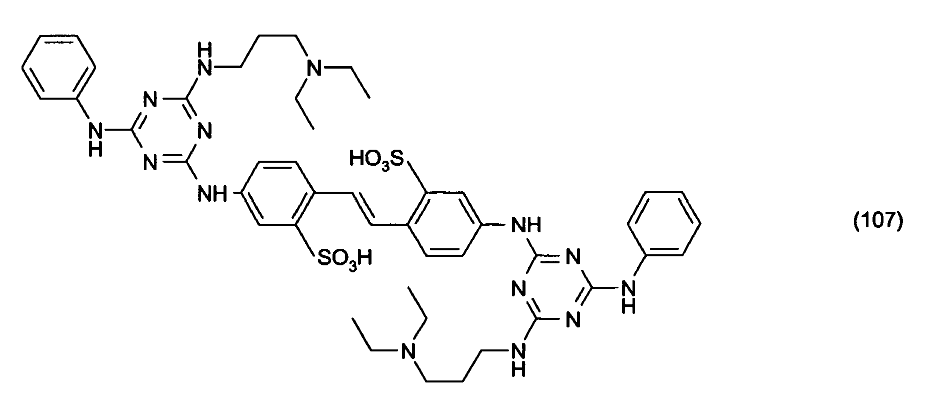

By proceeding essentially as described in Example 4, but replacing the 4,4'-bis [(4-N-morpholino-6-chloro-1,3,5-triazin-2-yl)amino]stilbene-2,2'-disulphonic acid disodium salt by an equivalent quantity of 4,4'-bis [(4-anilino-6-chloro-1,3,5-triazin-2-yl)amino]stilbene-2,2'-disulphonic acid disodium salt (106a) and the 2-N,N-diethylamino-1-ethylamine by an equivalent quantity of 3-N,N-diethylamino-1-propylamine, there are obtained 26.6g of the compound of formula (107) as beige crystals.

### Example 8

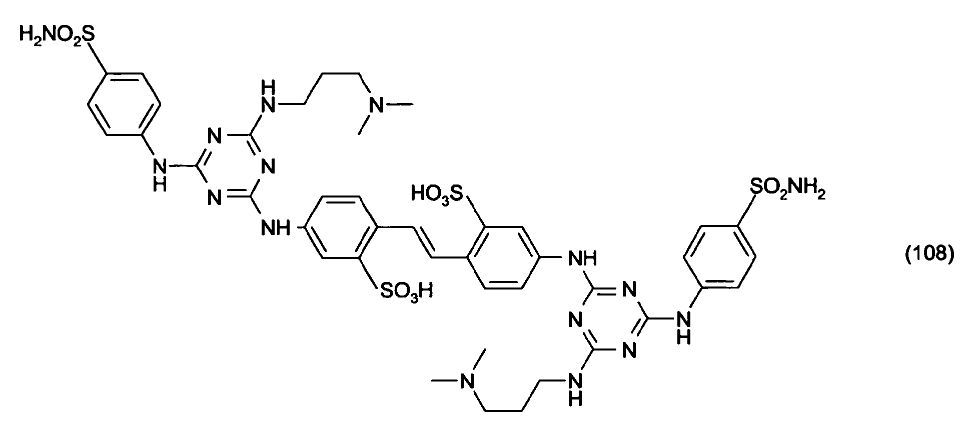

9.5g of 4,4'-bis {[4-(4-sulphonamidoanilino)-6-chloro-1,3,5-triazin-2-yl]amino}stilbene-2,2'-disulphonic acid disodium salt (108a), obtained by an analogous process to that described for compound (101a) in Example 1, are added over 20 minutes with stirring at 90°C to 32.0g of 3-N,N-dimethylamino-1-propylamine. The temperature is then further increased to 115-120°C and the mixture maintained at this temperature for a further 2 hours. Heating is then ceased, the mixture then diluted with 120ml of water and evaporated under vacuum. After repeating the latter procedure, the resulting residue is dissolved in 150ml of water and the pH adjusted to 12-13 by addition of aqueous sodium hydroxide. Subsequently, the pH is adjusted to 6 by addition of concentrated hydrochloric acid and the resulting precipitate filtered, washed with water and dried under vacuum at 70°C. There are obtained 8.4g of the compound of formula (108) as pale yellow crystals.

### Example 9

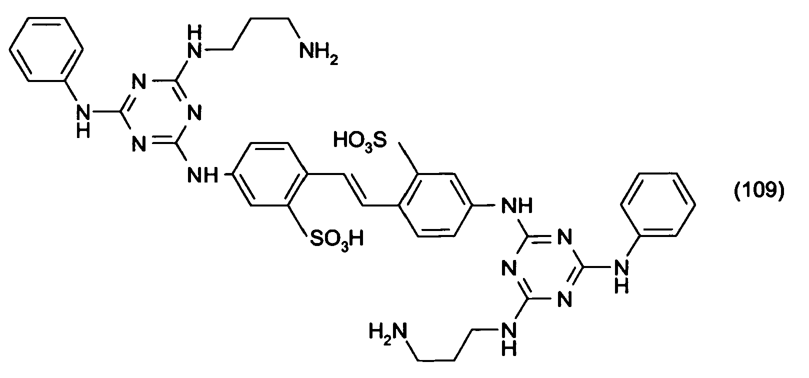

10.0g of 4,4'-bis [(4-anilino-6-chloro-1 ,3,5-triazin-2-yl)amino]stilbene-2,2'-disulphonic acid disodium salt (106a) are added over 15 minutes with stirring at 30°C to 30ml of 1,3-diaminopropane, whereby the temperature rises to 50°C. The yellow suspension is then heated to 80°C and stirring continued at this temperature for a further 90 minutes. After cooling, the mixture is poured into 300ml of water and the pH adjusted to 2 by addition of 65ml of concentrated hydrochloric acid. The aqueous liquors are decanted from the oily residue, which residue is ground with water in a mortar and then stirred for 2 hours at pH 5. The solids are filtered off, washed with 5% brine and dried under vacuum at 70°C. There are obtained 10.1g of the compound of formula (109) as yellow crystals.

### Example 10

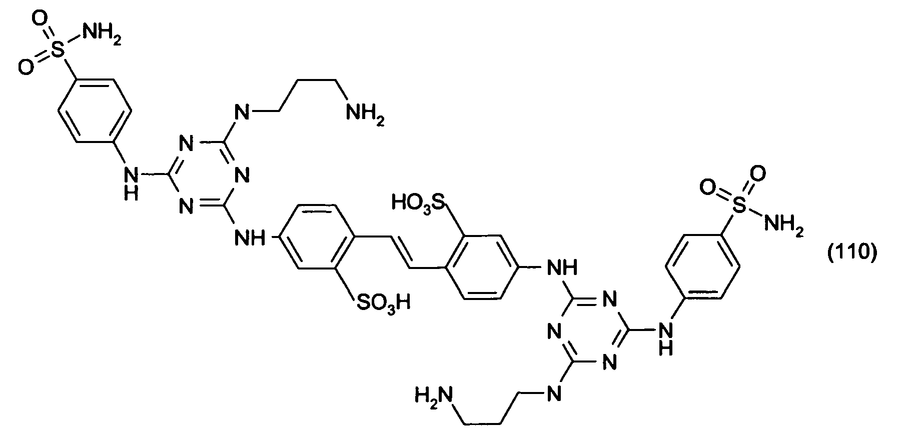

By following the procedure described in example 9, but replacing the 4,4'-bis [(4-anilino-6-chloro-1,3,5-triazin-2-yl)amino]stilbene-2,2'-disulphonic acid disodium salt by an equivalent quantity of 4,4'-bis {[4-(4-sulphonamidoanilino)-6-chloro-1,3,5-triazin-2-yl]amino}stilbene-2,2'-disulphonic acid disodium salt (108a), 12.0g of the compound of formula (110) are obtained as pale brown crystals.

### Example 11

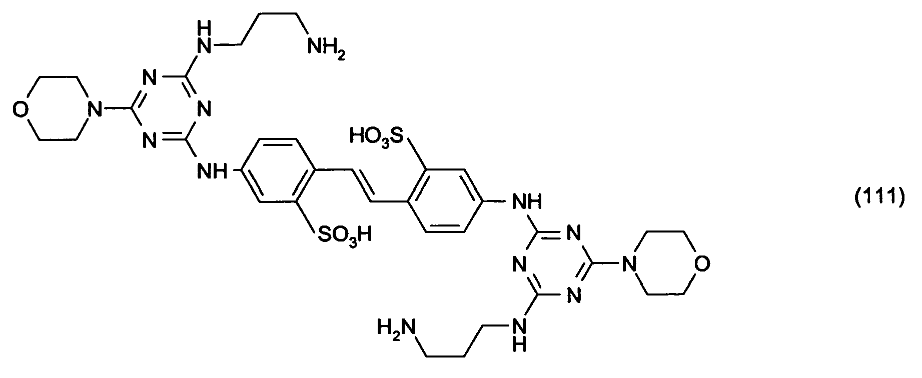

In a manner analogous to that described in Example 9, 8.5g of 4,4'-bis [(4-N-morpholino-6-chloro-1,3,5-triazin-2-yl)amino]stilbene-2,2'-disulphonic acid disodium salt (101a) are reacted with 30ml of 1,3-diaminopropane to yield 9.1g of the compound of formula (111) as yellow crystals.

### Example 12

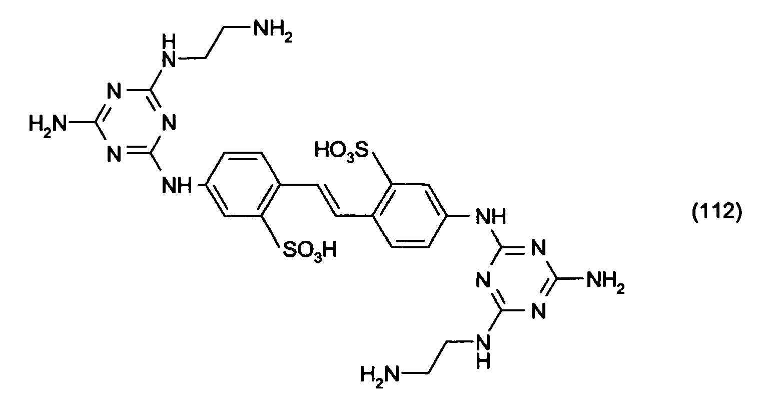

To a stirred mixture of 150ml of water, 150ml of dioxane and 40.7g of ethylene diamine, heated to 70-75°C, 40.0g of 4,4'-bis [(4-amino-6-chloro-1,3,5-triazin-2-yl)amino]stilbene-2,2'-disulphonic acid disodium salt are added over 30 minutes. The brown solution is then heated to 88°C and stirring continued for a further 2 hours. After cooling to 70°C, the pH is adjusted to 5.5 by addition of 115ml of concentrated hydrochloric acid and the precipitated solids filtered at 60°C and washed with a little water. The filter cake is suspended in 350ml of water, 50% aqueous sodium hydroxide solution added to pH 11 and the resulting yellow solution stirred for 1 hour. The pH is adjusted to 5 by addition of concentrated hydrochloric acid, the yellow precipitate filtered, washed with water and dried under vacuum at 70°C. There are obtained 31.5g of the compound of formula (112) as yellow crystals.

### Example 13

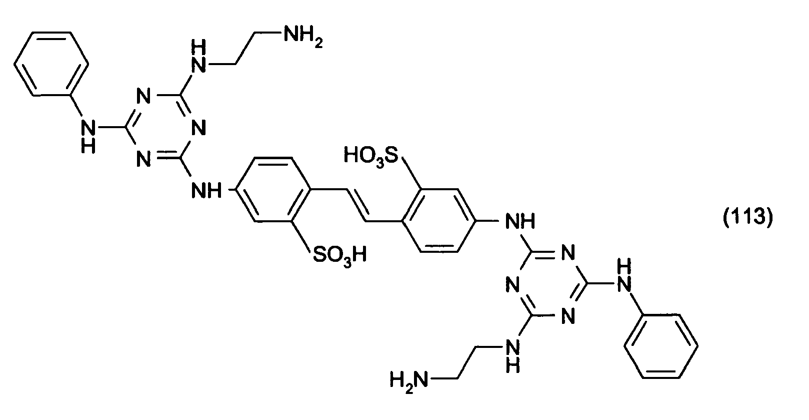

By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 40.0g of 4,4'-bis [(4-anilino-6-chloro-1,3,5-triazin-2-yl)amino]stilbene-2,2'-disulphonic acid disodium salt (106a), there are obtained 30.4g of the compound of formula (113) as yellow crystals.

### Example 14

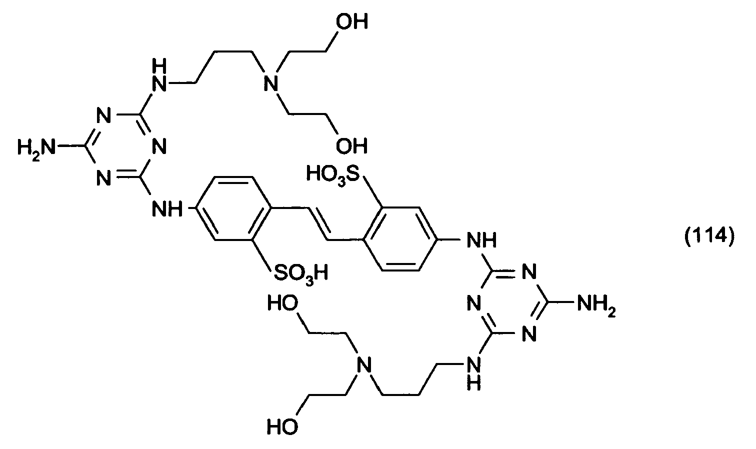

By proceeding essentially as described in Example 12, but replacing the 40.7g of ethylene diamine by 91.6g of N-(3-aminopropyl)diethanolamine, there are obtained 50.4g of the compound of formula (114) as yellow crystals.

### Example 15

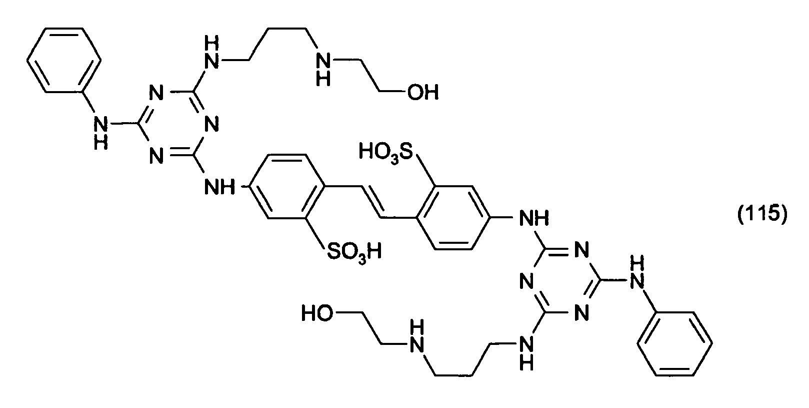

By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 40.0g of 4,4'-bis [(4-anilino-6-chloro-1,3,5-triazin-2-yl)amino]stilbene-2,2'-disulphonic acid disodium salt (106a) and the 40.7g of ethylene diamine by 68.1g of 2-(3-aminopropylamino) ethanol, there are obtained 35.8g of the compound of formula (115) as yellow crystals.

### Example 16

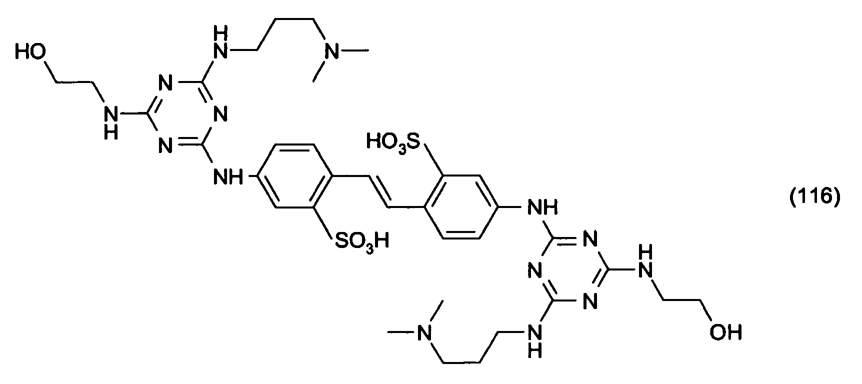

By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 35.0g of 4,4'-bis [(4-ethanolamino-6-chloro-1,3,5-triazin-2-yl)amino]stilbene-2,2'-disulphonic acid disodium salt and the 40.7g of ethylene diamine by 47.0g of 3-N,N-dimethylamino-1-propylamine, there are obtained 39.3g of the compound of formula (116) as yellow crystals.

### Example 17

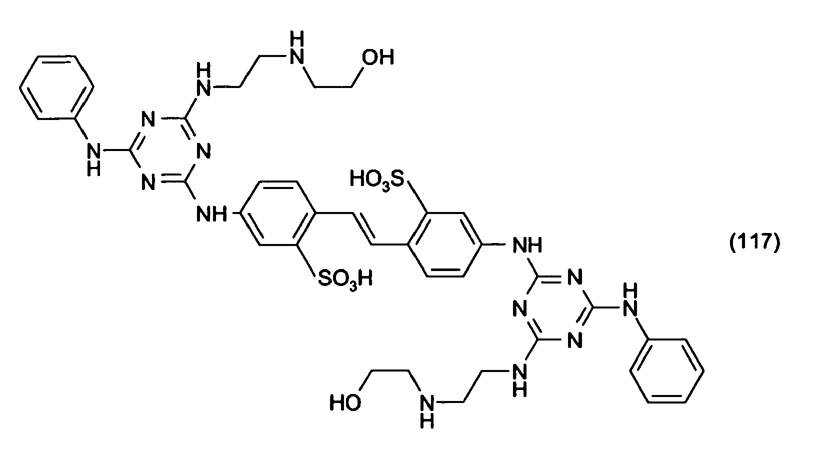

Treatment of 30.0g of 4,4'-bis [(4-anilino-6-chloro-1,3,5-triazin-2-yl)amino]stilbene-2,2'-disulphonic acid disodium salt (106a) with 90ml of N-(2-hydroxyethyl) ethylene diamine, essentially as described in Example 9, results in 28.0g of the compound of formula (117) as beige crystals.

### Example 18

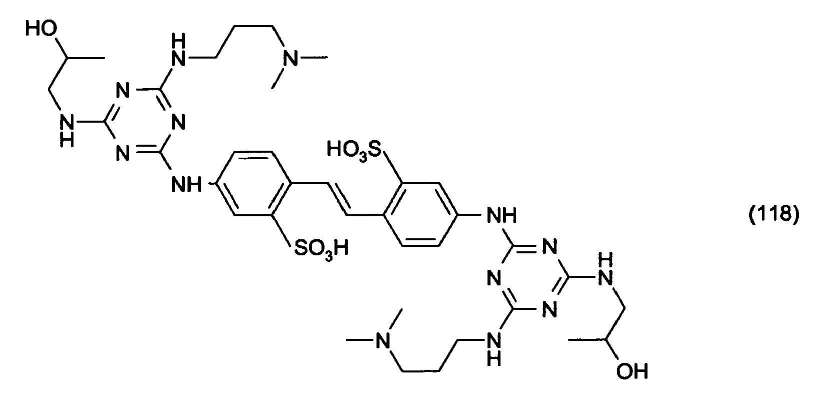

A solution of 120g of cyanuric chloride in 930ml of methyl ethyl ketone is added with stirring over 10 minutes at 5-10°C to 400g of ice/water. Then, during 70 minutes at a pH of from 4.5 to 5.0, 1083g of a 12% solution of 4,4'-diaminostilbene-2,2'-disulphonic acid and sodium carbonate are added such that no excess of 4,4'-diaminostilbene-2,2'-disulphonic acid is present. The mixture is stirred for a further 10 minutes at 5-10°C, after which time a total of 29.8ml of 20% aqueous sodium carbonate solution is consumed. The mixture is warmed to 10-20°C and the pH adjusted to 7.0-7.5 by addition of 50% aqueous sodium hydroxide solution. 52.5g of 1-amino propan-2-ol are then added drop wise over 10 minutes, the mixture warmed to 70°C over 1 hour and stirring continued for a further 90 minutes at this temperature, the methyl ethyl ketone being distilled off, then cooled to 50°C during 30 minutes, then to 25°C during a further 30 minutes, stirred for a further 3 hours at this temperature and, finally, allowed to stand overnight at room temperature. The pH is maintained at 7.0-7.5 during the entire period, whereby a total of 53.4ml of a solution of 50% aqueous sodium hydroxide solution is consumed. The precipitated solids are filtered washed with water, then with 2.5% brine and dried under vacuum at 70°C. There are obtained 230.6g of the compound of formula 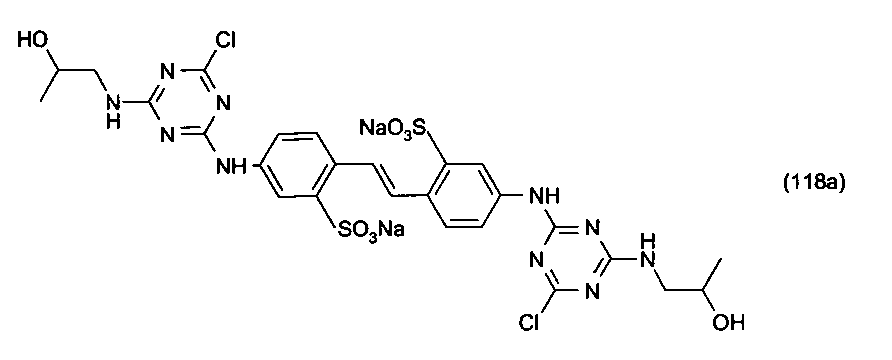 as yellow crystals.

To a mixture of 150ml of water, 150ml of dioxane and 43.1g of 3-N,N-dimethylamino-1-propylamine, previously warmed to 70°C, 35.0g of the compound of formula (118a) are added with stirring. The yellowish brown solution is warmed to 86-88°C and stirring continued for 90 minutes at this temperature. After cooling to 70°C, 100ml of water are added and the pH adjusted to 5.0 by addition of 70ml of concentrated hydrochloric acid. After adjusting the pH to 1.5 and cooling to 10°C, 25g of sodium chloride are added and the mixture stirred overnight. The mixture is then evaporated on a rotary evaporator and the resulting viscous residue added in portions to 400ml of acetone. The supernatant liquids are discarded and the procedure repeated until a crystalline product results. After filtering, the solids are stirred overnight in 200ml of water, the supernatant liquids discarded, the residue evaporated on a rotary evaporator and finally dried under vacuum at 70°C. There are obtained 13.Og of the compound of formula (118) as pale yellow crystals.

### Example 19

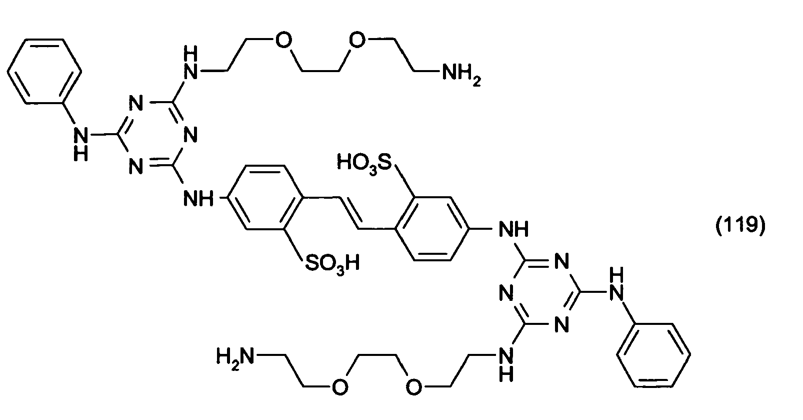

Treatment of 30.0g of 4,4'-bis [(4-anilino-6-chloro-1,3,5-triazin-2-yl)amino]stilbene-2,2'-disulphonic acid disodium salt (106a) with 100ml of 2,2'-(ethylenedioxy)-diethylene diamine, essentially as described in Example 9, results in 34.0g of the compound of formula (119) as pale brown crystals.

### Example 20

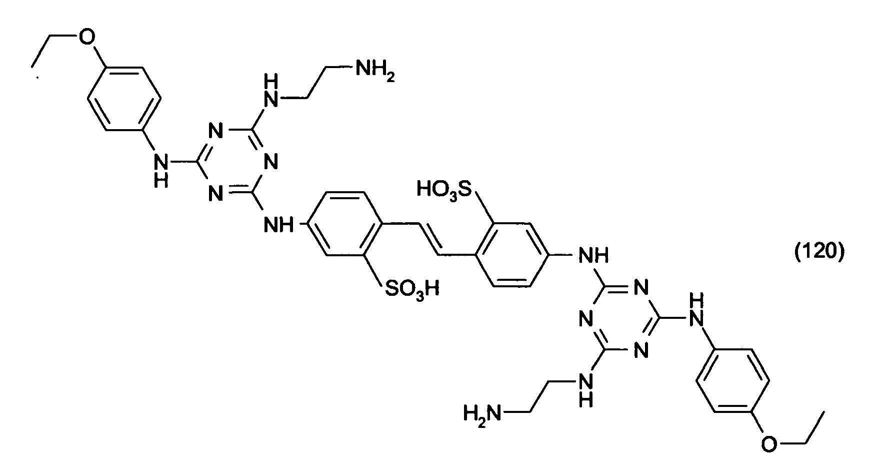

By following the procedure described in Example 1 for the preparation of the compound of formula (101a), but replacing the morpholine by an equivalent quantity of p-phenetidine, there are obtained 232.7g of the compound of formula 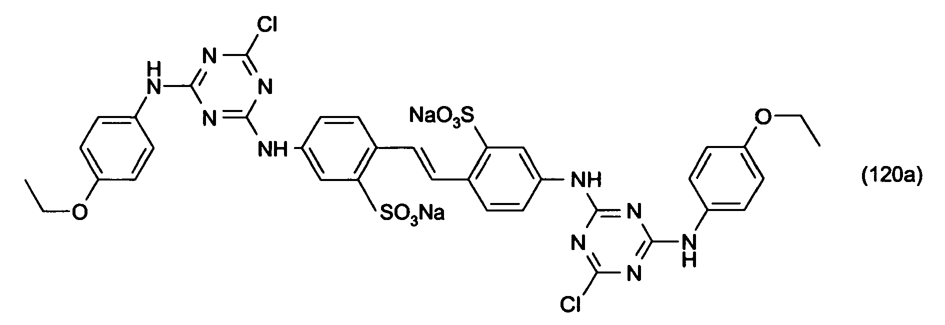 as greenish yellow crystals. By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 35.0g of the compound of formula (120a), 27.2g of the compound of formula (120) are obtained as yellow crystals.

### Example 21

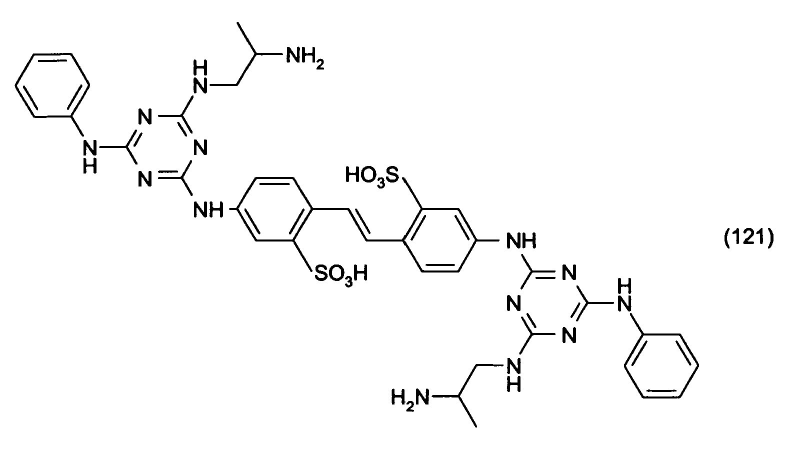

By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 40.0g of 4,4'-bis [(4-anilino-6-chloro-1,3,5-triazin-2-yl)amino]stilbene-2,2'-disulphonic acid disodium salt (106a) and the 40.7g of ethylene diamine by 35.5g of 1,2-propylene diamine, there are obtained 34.7g of the compound of formula (121) as yellow crystals.

### Example 22

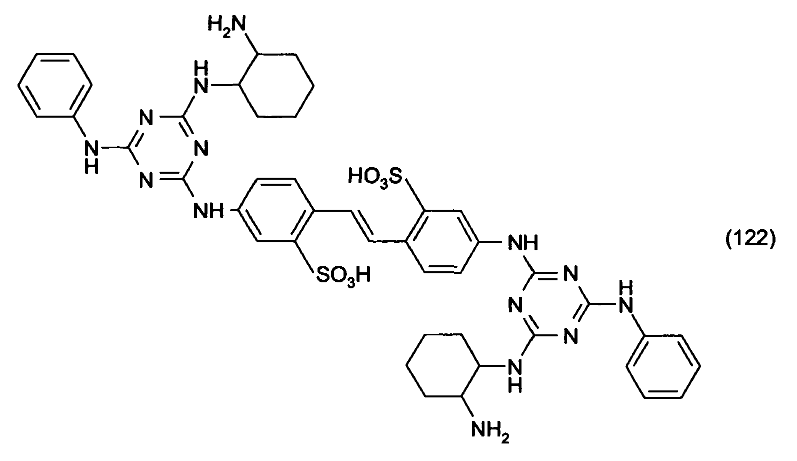

By proceeding essentially as described in Example 12, but replacing the 40.0g of 4,4'-bis [(4-amino-6-chloro-1,3,5-triazin-2-yl)amino]stilbene-2,2'-disulphonic acid disodium salt by 30.0g of 4,4'-bis [(4-anilino-6-chloro-1,3,5-triazin-2-yl)amino]stilbene-2,2'-disulphonic acid disodium salt (106a) and the 40.7g of ethylene diamine by 48.3g of 1,2-diaminocyclohexane, there are obtained 27.1g of the compound of formula (122) as yellow crystals.

### Example 23

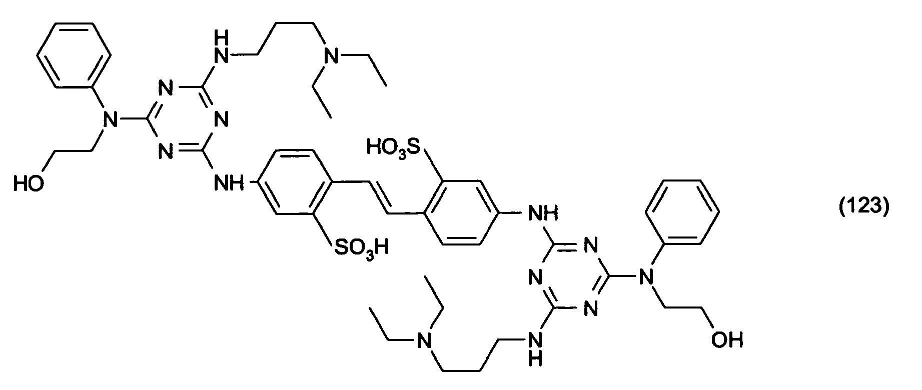

By proceeding essentially as described in Example 18, but replacing the 52.5g of 1-amino propan-2-ol by 89.1g of 2-anilinoethanol, there are obtained 281.5g of the compound of formula 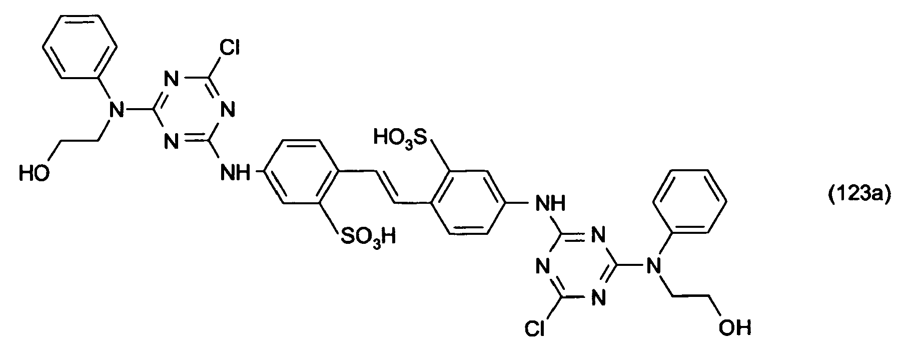 as yellow crystals.

To 150ml of water, previously warmed to 70-75°C, 35.0g of the compound of formula (123a) are added. The resulting yellow solution is then treated with 12.6g of 3-N,N-diethylamino-1-propylamine and the mixture stirred for 4 hours at 95-97°C, the pH being maintained at 10.0-10.5 by addition of a total of 1.5ml of 4N aqueous sodium hydroxide solution. After cooling to 70°C, the pH is adjusted to 4.0 by addition of 6.5ml of concentrated hydrochloric acid and the precipitated solids filtered, washed with water and dried under vacuum at 80°C. There are obtained 37.4g of the compound of formula (123) as yellow crystals.

### Example 24

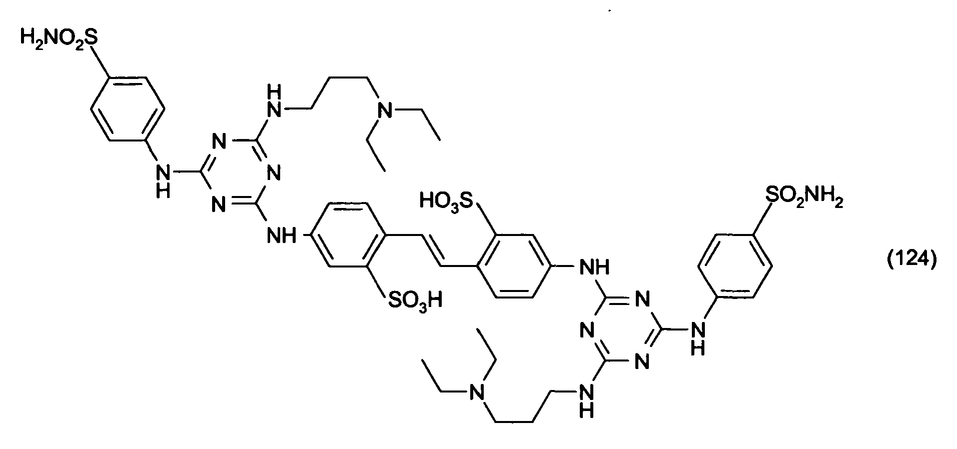

Treatment of 25.0g of 4,4'-bis {[4-(4-sulphonamidoanilino)-6-chloro-1,3,5-triazin-2-yl]aminolstilbene-2,2'-disulphonic acid disodium salt (108a) with 9.6g of 3-diethylamino-1-propylamine by an analogous process to that described for compound (123) in the previous example, results in the formation of 24.0g of the compound of formula (124) as yellow crystals.

### Example 25

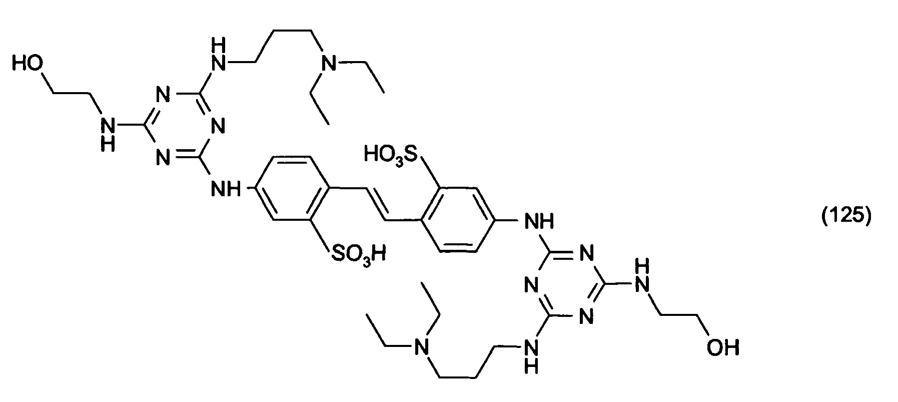

By proceeding essentially as described in Example 16, but replacing the 3-N,N-dimethylamino-1-propylamine by 3-N,N-diethylamino-1-propylamine, there are obtained 40.4g of the compound of formula (125) as yellow crystals.

### Example 26

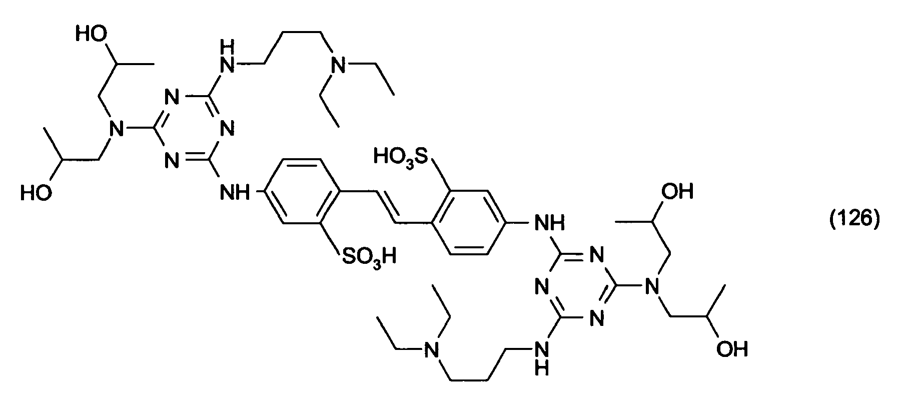

Treatment of 65.2g of 4,4'-bis [(4-bis (2-hydroxy-n-propyl)amino-6-chloro-1,3,5-triazin-2-yl)amino]stilbene-2,2'-disulphonic acid disodium salt (103a) with 13.9g of 3-diethylamino-1-propylamine by an analogous process to that described for compound (123) in Example 23, results in the formation of 28.8g of the compound of formula (126) as yellow crystals.

### Example 27

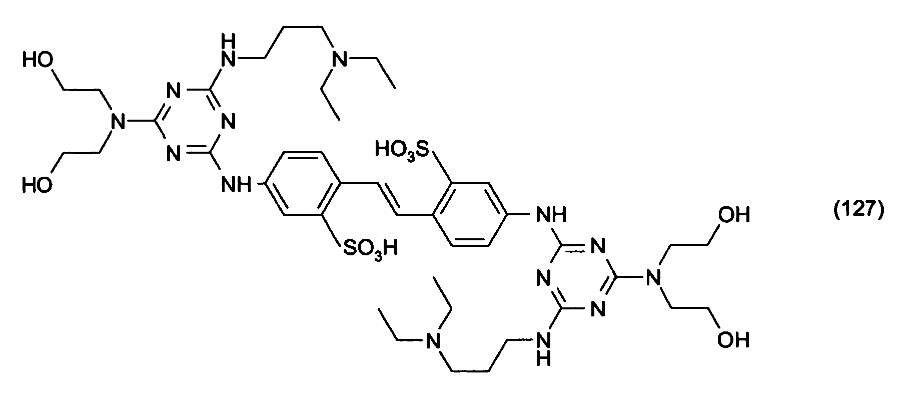

Treatment of 25g of 4,4'-bis [(4-bis-(2-hydroxethyl)amino-6-chloro-1,3,5-triazin-2-yl)amino]stilbene-2,2'-disulphonic acid disodium salt (102a) with 11.6g of 3-diethylamino-1-propylamine by an analogous process to that described for compound (123) in Example 23, results in the formation of 24.1 g of the compound of formula (127) as yellow crystals.

### Example 28

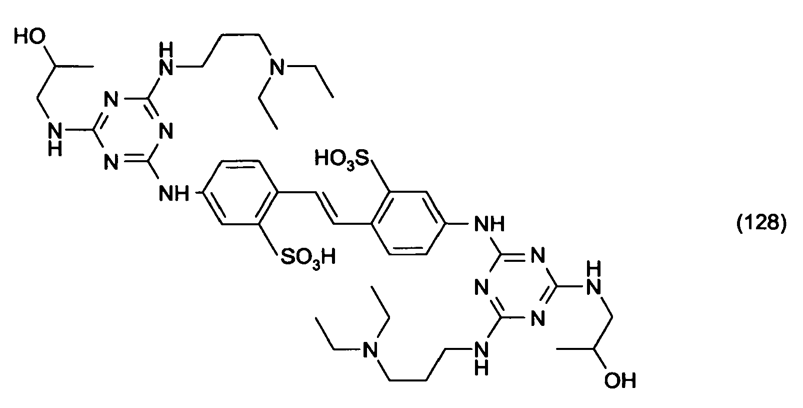

Treatment of 25g of the compound of formula (118a) with 9.95g of 3-diethylamino-1-propylamine by an analogous process to that described for compound (129) in Example 23, results in the formation of 24.6g of the compound of formula (128) as yellow crystals.

### Example 29

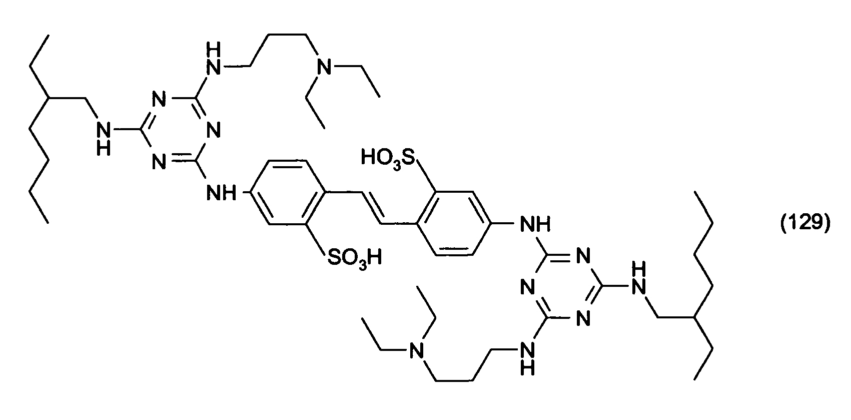

By proceeding essentially as described in Example 18, but replacing the 52.5g of 1-amino-propan-2-ol by 84.0g of 2-ethyl-1-hexylamine, there are obtained 270.7g of the compound of formula 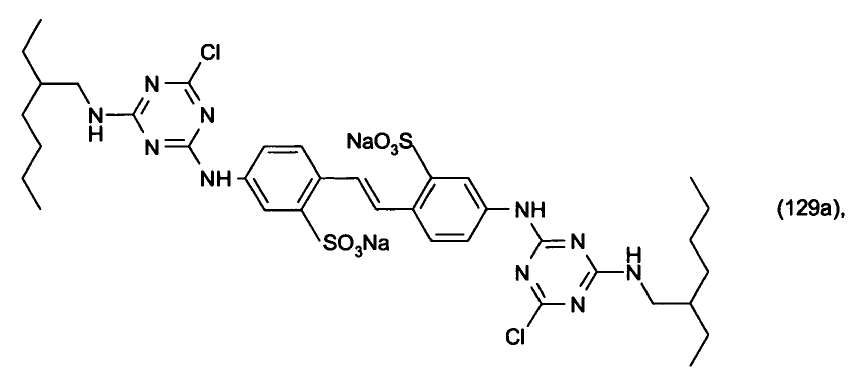 as yellowish beige crystals.

Treatment of 25.0g of the compound of formula (129a) with 10.5g of 3-diethylamino-1-propylamine by an analogous process to that described for compound (129) in Example 23, results in the formation of 26.7g of the compound of formula (129) as pale yellow crystals.

### Example 30

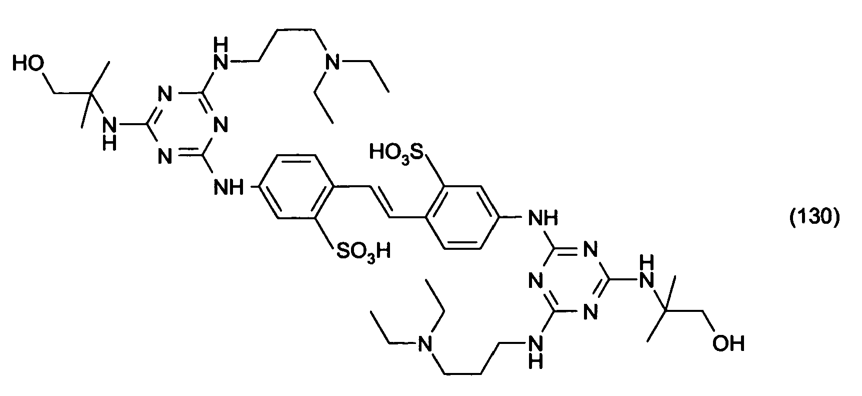

By proceeding essentially as described in Example 18, but replacing the 52.5g of 1-amino-propan-2-ol by 64.3g of 2-amino-2-methyl-1-propanol, there are obtained 162.4g of the compound of formula 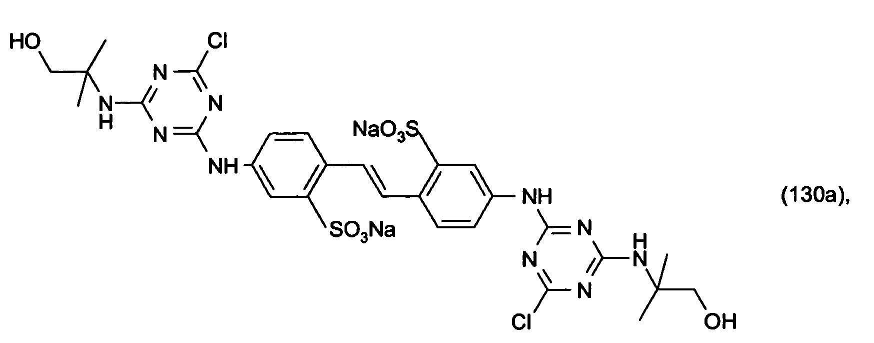 as yellow crystals.

Treatment of 25g of the compound of formula (130a) with 11.6g of 3-diethylamino-1-propylamine by an analogous process to that described for compound (123) in Example 23, results in the formation of 29.2g of the compound of formula (130) as beige crystals.

### Example 31

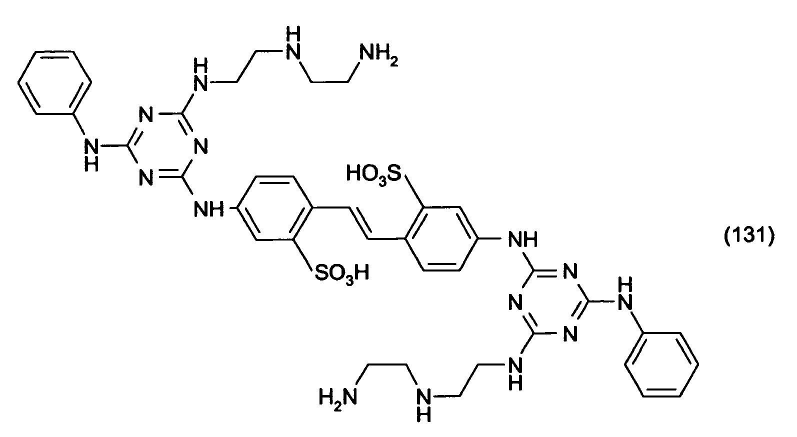

By proceeding essentially as described in Example 15, but replacing the 40.0g of 4,4'-bis [(4-amino-6-chloro-1,3,5-triazin-2-yl)amino]stilbene-2,2'-disulphonic acid disodium salt by 35.0g of 4,4'-bis [(4-anilino-6-chloro-1,3,5-triazin-2-yl)amino]stilbene-2,2'-disulphonic acid disodium salt (106a) and the 40.7g of ethylene diamine by 44.6g of diethylene triamine, there are obtained 37.1g of the compound of formula (131) as yellow crystals.

### Application Examples

The various fluorescent whitening agents (FWA's) are dissolved in 25ml of a 9:1 mixture of dimethyl sulphoxide/water, the pH adjusted to approximately 10 by addition of 4N aqueous sodium hydroxide solution and the solutions made up to 50ml with water.

To a fibre dispersion consisting of 70 parts birch and 30 parts pine Kraft fibre with a degree of refining of 35° SR, 10% calcium carbonate (Hydrocarb 60) is added as filler. Sufficient of the FWA solutions are then added such that the FWA concentration, based on the weight of the pulp fibre, is 0.2%. The FWA is allowed to exhaust for 15 minutes, 0.03% of a cationic polyacrylamide (Percol 292) added as retention auxiliary and the hand sheet formed immediately by means of the Rapid-Koethen system.

The degrees of whiteness of the sheets (W CIE) are then measured by SCAN-P66-93 using a spectrophotometer.

The results of the measurements are summarized in the following Table 1.

**Table 1**

| **Example Nr.** | **Compound Nr.** | **W (CIE)** |
|---|---|---|
| | None | 70.1 |
| 32 | (121) | 132 |
| 33 | (109) | 131 |
| 34 | (112) | 130 |
| 35 | (111) | 125 |
| 36 | (119) | 124 |
| 37 | (101) | 115 |
| 38 | (102) | 113 |
| 39 | (120) | 112 |

The above results clearly demonstrate the excellent whitening effects of the fluorescent whitening agent compositions of the invention.

## Claims

1. Use of the compound of formula 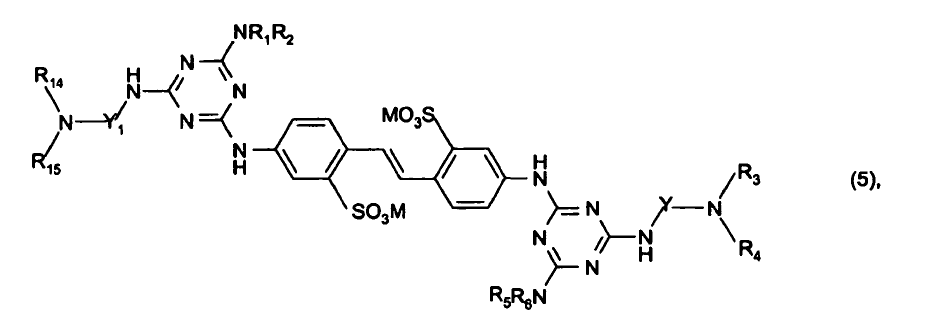 in which
Y and Y₁ each, independently of each other, represent a straight chain C₂-C₈alkylene or branched C₃-C₈alkylene residue, which may be interrupted by one or two nitrogen, oxygen or sulphur atoms or represent a 5- or 6-membered cycloaliphatic ring,
R₁, R₂, R₅ and R₈ each, independently of each other, represent hydrogen, C₁-C₈alkyl, C₂-C₄hydroxyalkyl, C₁-C₄alkoxyC₁-C₄alkyl, phenyl, which is unsubstituted or substituted by halogen, C₁-C₄alkoxy, C₁-C₄alkyl or sulphonamido or
R₁ and R₂ and/or R₅ and R₆, together with the nitrogen atom to which they are attached, complete a morpholino-, piperidino- or pyrrolidino-ring,
R₃, R₄, R₁₄ and R₁₅, each independently of each other, represent hydrogen, C₁-C₄alkyl or C₂-C₄hydroxyalkyl or
R₃ and R₄, together with the nitrogen atom to which they are attached, complete a morpholino-, piperidino- or pyrrolidino-ring and
M represents hydrogen, an alkaline or alkaline earth metal, ammonium or alkylammonium, for the fluorescent whitening of paper.

2. Use, according to claim 1, whereby, in the compound of formula (5),
Y and Y₁ both represent a straight chain C₂-C₆alkylene residue, which may be interrupted by one nitrogen or two oxygen atoms, a branched C₃-C₆alkylene residue or a 1,2-substituted cylohexyl moiety,
R₁, R₂, R₅ and R₆ each represent hydrogen, C₁-C₈alkyl, C₂-C₄hydroxyalkyl, phenyl, which is unsubstituted or substituted by C₁-C₂alkoxy or sulphonamido or
R₁ and R₂ and/or R₅ and R₆, together with the nitrogen atom to which they are attached, complete a morpholine ring,
R₃, R₄, R₁₄ and R₁₅, each represent hydrogen, C₁-C₄alkyl or C₂-C₄hydroxyalkyl and
M represents hydrogen.

3. A composition for whitening paper, which comprises water, a fluorescent whitening agent of formula (5), according to claim 1 or claim 2 and, optionally, further auxiliaries.

4. Paper, which has been treated with a fluorescent whitening agent of formula (5), according to claim 1 or claim 2, or with a composition, according to claim 3.

5. A method for the fluorescent whitening of paper, comprising contacting the paper with a fluorescent whitening agent which comprises the compound of formula (5) according to claim 1 or 2.

## Patentansprüche

1. Verwendung der Verbindung der Formel 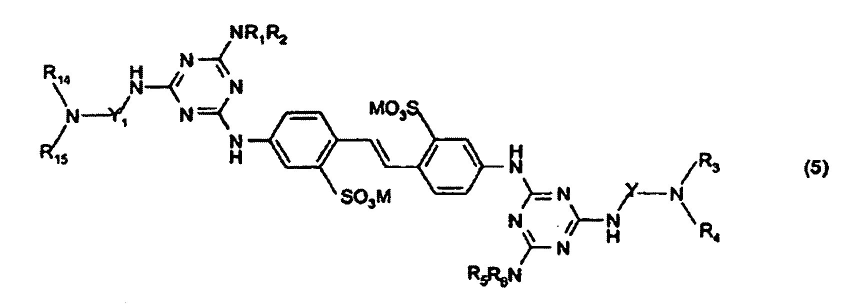 worin
Y und Y₁ jeweils unabhängig voneinander einen gerad-kettigen C₂-C₈-Alkylen- oder verzweigten C₃-C₈-Alkylen-Rest, der durch ein oder zwei Stickstoff-, Sauerstoff- oder Schwefel-Atome unterbrochen sein kann, wiedergeben oder einen 5- oder 6-gliedrigen cycloaliphatischen Ring wiedergeben,
R₁, R₂, R₅ und R₆ jeweils unabhängig voneinander Wasserstoff, C₁-C₈-Alkyl, C₂-C₄-Hydroxyalkyl, C₁-C₄-Alkoxy-C₁-C₄-alkyl, Phenyl, das unsubstituiert oder mit Halogen, C₁-C₄-Alkoxy, C₁-C₄-Alkyl oder Sulfonamido substituiert ist, wiedergeben oder
R₁ und R₂ und/oder R₅ und R₆ zusammen mit dem Stickstoff-Atom, an das sie gebunden sind, einen Morpholino-, Piperidino- oder Pyrrolidino-Ring vervollständigen,
R₃, R₄, R₁₄ und R₁₅ jeweils unabhängig voneinander Wasserstoff, C₁-C₄-Alkyl oder C₂-C₄-Hydroxyalkyl wiedergeben oder
R₃ und R₄ zusammen mit dem Stickstoff-Atom, an das sie gebunden sind, einen Morpholino-, Piperidino- oder Pyrrolidino-Ring vervollständigen und
M Wasserstoff, ein Alkali- oder Erdalkalimetall, Ammonium oder Alkylammonium wiedergibt,
für das fluoreszierende Weißen bzw. optische Bleichen bzw. optische Aufhellen von Papier.

2. Verwendung nach Anspruch 1, wobei in der Verbindung der Formel (5),
Y und Y₁ beide einen gerad-kettigen C₂-C₆-Alkylen-Rest, der durch ein Stickstoff- oder zwei Sauerstoff-Atome unterbrochen sein kann, einen verzweigten C₃-C₆-Alkylen-Rest oder eine 1,2-substituierte Cylohexyl-Einheit wiedergeben,
R₁, R₂, R₅ und R₆ jeweils Wasserstoff, C₁-C₈-Alkyl, C₂-C₄-Hydroxyalkyl, Phenyl, das unsubstituiert oder mit C₁-C₂ Alkoxy oder Sulfonamido substituiert ist, wiedergeben oder
R₁ und R₂ und/oder R₅ und R₆ zusammen mit dem Stickstoff-Atom, an das sie gebunden sind, einen Morpholin-Ring vervollständigen,
R₃, R₄, R₁₄ und R₁₅ jeweils Wasserstoff, C₁-C₄-Alkyl oder C₂-C₄-Hydroxyalkyl wiedergeben und
M Wasserstoff wiedergibt.

3. Zusammensetzung zum Weißen von Papier, die Wasser, einen fluoreszierenden Weißmacher bzw. ein optisches Bleichmittel bzw. einen optischen Aufheller der Formel (5) nach Anspruch 1 oder Anspruch 2 und gegebenenfalls weitere Hilfsmittel umfasst.

4. Papier, das mit einem fluoreszierenden Weißmacher bzw. optischen Bleichmittel bzw. einen optischen Aufheller der Formel (5) nach Anspruch 1 oder Anspruch 2 oder mit einer Zusammensetzung nach Anspruch 3 behandelt wurde.

5. Verfahren zum fluoreszierenden Weißen bzw. optischen Bleichen bzw. optischen Aufhellen von Papier, umfassend in Kontakt bringen des Papiers mit einem fluoreszierenden Weißmacher bzw. optischen Bleichmittel bzw. optischen Aufheller, das die Verbindung der Formel (5) nach Anspruch 1 oder 2 umfasst.

## Revendications

1. Utilisation du composé de formule 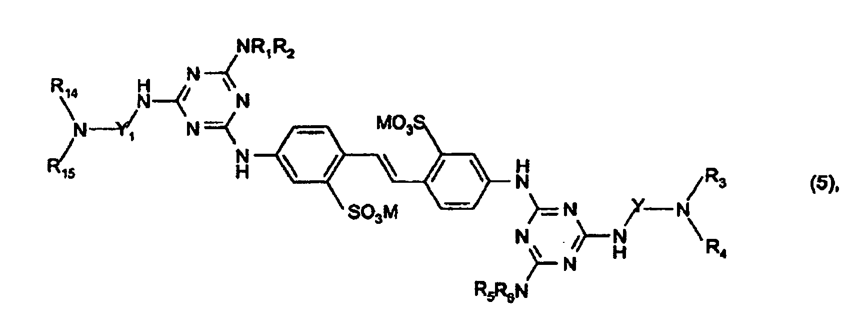 dans lequel
Y et Y₁ représentent chacun, indépendamment l'un de l'autre, un résidu d'alkylène en C₂-C₈ à chaîne linéaire ou d'alkylène en C₃-C₈ ramifié, qui peut être interrompu par un ou deux atomes d'azote, d'oxygène ou de soufre ou représentent un cycle cycloaliphatique à 5 ou 6 chaînons,
R₁, R₂, R₅ et R₆ représentent chacun, indépendamment les uns des autres, un hydrogène, un alkyle en C₁-C₈, un hydroxyalkyle en C₂-C₄, un alcoxy en C₁-C₄-alkyle en C₁-C₄, un phényle, qui est non substitué ou substitué par un halogène, un alcoxy en C₁-C₄, un alkyle en C₁-C₄ ou un sulfonamido ou
R₁ et R₂ et/ou R₅ et R₆, conjointement avec l'atome d'azote auquel ils sont liés, complètent un cycle morpholino, pipéridino ou pyrrolidino,
R₃, R₄, R₁₄ et R₁₅ représentent, chacun indépendamment les uns des autres, un hydrogène, un alkyle en C₁-C₄ ou un hydroxyalkyle en C₂-C₄ ou
R₃ et R₄, conjointement avec l'atome d'azote auquel ils sont liés, complètent un cycle morpholino, pipéridino ou pyrrolidino et
M représente un hydrogène, un métal alcalin ou alcalino-terreux, l'ammonium ou l'alkylammonium,
pour le blanchiment de papier par agent fluorescent.

2. Utilisation, selon la revendication 1, au moyen de laquelle, dans le composé de formule (5),
Y et Y₁ représentent tous les deux un résidu d'alkylène en C₂-C₆ à chaîne linéaire, qui peut être interrompu par un atome d'azote ou deux atomes d'oxygène, un résidu d'alkylène en C₃-C₆ ramifié ou un fragment de cyclohexyle 1,2-substitué,
R₁, R₂, R₅ et R₆ représentent chacun un hydrogène, un alkyle en C₁-C₈, un hydroxyalkyle en C₂-C₄, un phényle, qui est non substitué ou substitué par un alcoxy en C₁-C₂ ou un sulfonamido ou
R₁ et R₂ et/ou R₅ et R₆, conjointement avec l'atome d'azote auquel ils sont liés, complètent un cycle morpholine,
R₃, R₄, R₁₄ et R₁₅ représentent chacun un hydrogène, un alkyle en C₁-C₄ ou un hydroxyalkyle en C₂-C₄ et
M représente un hydrogène.

3. Composition de blanchiment pour le papier, qui comprend de l'eau, un agent de blanchiment fluorescent de formule (5), selon la revendication 1 ou la revendication 2 et, éventuellement, d'autres agents auxiliaires.

4. Papier, qui a été traité avec un agent de blanchiment fluorescent de formule (5), selon la revendication 1 ou la revendication 2, ou avec une composition, selon la revendication 3.

5. Procédé de blanchiment de papier par agent fluorescent, comprenant la mise en contact du papier avec un agent de blanchiment fluorescent qui comprend le composé de formule (5) selon la revendication 1 ou 2.
